# EUROPEAN PATENT APPLICATION

(11) **EP 3 988 180 A1**
(43) Date of publication of application: **27.04.2022**
(21) Application number: 20826105.7
(22) Date of filing: 19.06.2020
(51) Int. Cl.: A62D 1/00, C07C 21/18, C07C 17/42, C09K 3/00, C09K 3/30, C11D 7/22, C11D 7/26, C11D 7/30, F25B 1/00, C09K 5/04

(54) **FLUOROETHYLENE COMPOSITION**

(30) Priority: 19.06.2019 JP 2019113883
(71) Applicant: Daikin Industries, Ltd., Osaka-shi, Osaka 530-8323 (JP)
(72) Inventor: GOTOU, Tomoyuki, Osaka-shi, Osaka 530-8323 (JP); OHKUBO, Shun, Osaka-shi, Osaka 530-8323 (JP); WATANABE, Yuka, Osaka-shi, Osaka 530-8323 (JP)
(74) Representative: Hoffmann Eitle
(86) International application number: PCT/JP2020/024261
(87) International publication number: WO 2020/256146

(57) **Abstract**

Provided is a composition that contains a fluoroethylene having one or more fluorine atoms, the composition having excellent stability of the fluoroethylene. The fluoroethylene composition of the present disclosure contains a fluoroethylene having one or more fluorine atoms, water, and oxygen, the composition having a water content of 100 mass ppm or less based on the mass of the fluoroethylene, and an oxygen content of 0.35 mol% or less based on the fluoroethylene. The fluoroethylene composition has excellent stability of fluoroethylene.

## Description

### Technical Field

The present disclosure relates to a fluoroethylene composition.

### Background Art

Fluoroethylenes, which contain one or more fluorine atoms, are used for various applications. In particular, 1,2-difluoroethylene (HFO-1132) is known to be usable as a working medium for heat cycle (see, for example, Patent Literature (PTL) 1) .

### Citation List

### Patent Literature

PTL 1: WO 2012/157765

### Summary of Invention

### Technical Problem

An object of the present disclosure is to provide a composition containing a fluoroethylene having one or more fluorine atoms, the composition having excellent stability of the fluoroethylene.

### Solution to Problem

The present disclosure includes the embodiments described in the following items.

### Item 1.

A fluoroethylene composition containing a fluoroethylene having one or more fluorine atoms, water, and oxygen, the composition having a water content of 100 mass ppm or less based on the mass of the fluoroethylene, and an oxygen content of 0.35 mol% or less based on the fluoroethylene.

### Item 2.

The fluoroethylene composition according to Item 1, wherein the fluoroethylene is at least one member selected from the group consisting of 1,2-difluoroethylene and trifluoroethylene.

### Item 3.

The fluoroethylene composition according to Item 2, wherein the fluoroethylene is 1,2-difluoroethylene.

### Item 4.

The fluoroethylene composition according to Item 2 or 3, wherein the 1,2-difluoroethylene is trans-1,2-difluoroethylene (HFO-1132(E)) and/or cis-1,2-difluoroethylene (HFO-1132(Z)).

### Item 5.

The fluoroethylene composition according to any one of Items 1 to 4, further comprising at least one member selected from the group consisting of polyalkylene glycols, polyol esters, and polyvinyl ethers.

### Item 6.

The fluoroethylene composition according to any one of Items 1 to 5, which is used for a heat transfer medium, a refrigerant, a foaming agent, a solvent, a cleaning agent, a propellant, or a fire extinguisher.

### Item 6-1.

A heat transfer medium, a refrigerant, a foaming agent, a solvent, a cleaning agent, a propellant, or a fire extinguisher containing the fluoroethylene composition of any one of Items 1 to 5.

### Item 6-2.

Use of the fluoroethylene composition of any one of Items 1 to 5 for a heat transfer medium, a refrigerant, a foaming agent, a solvent, a cleaning agent, a propellant, or a fire extinguisher.

### Item 7.

A method for stabilizing a fluoroethylene composition containing a fluoroethylene having one or more fluorine atoms, water, and oxygen, the composition having an oxygen content of 0.35 mol% or less based on the fluoroethylene, the method comprising adjusting the content of the water to 100 mass ppm or less based on the mass of the fluoroethylene.

### Advantageous Effects of Invention

The fluoroethylene composition of the present disclosure exhibits excellent stability of fluoroethylene.

### Description of Embodiments

The present inventors conducted extensive research to improve the stability of fluoroethylene, and found that the above object can be achieved when water coexists with fluoroethylene. Below, embodiments encompassed by the present disclosure will be described in detail. In the present specification, the terms "comprise" and "contain" include the concepts of comprising, containing, consisting essentially of, and consisting of.

The numerical range expressed by using the term "to" in the present specification indicates a range that includes numerical values shown before and after "to" as the minimum and maximum values, respectively. In the numerical ranges stated in steps in the present specification, the upper-limit value or the lower-limit value of one numerical range can be randomly combined with the upper-limit value or the lower-limit value of another numerical range. The upper-limit values or the lower-limit values of the numerical ranges stated in the present specification may be replaced with a value shown in the Examples or a value that can be unambiguously derived from the Examples.

The fluoroethylene composition of the present disclosure contains a fluoroethylene having one or more fluorine atoms, water, and oxygen, the composition having a water content of 100 mass ppm or less based on the mass of the fluoroethylene, and an oxygen content of 0.35 mol% or less based on the fluoroethylene. Below, the fluoroethylene having one or more fluorine atoms in the fluoroethylene composition of the present disclosure is abbreviated as "fluoroethylene A."

Fluoroethylene A preferably has two or more fluorine atoms, more preferably two or three fluorine atoms, and particularly preferably two fluorine atoms.

Fluoroethylene A is preferably at least one member selected from the group consisting of 1,2-difluoroethylene and trifluoroethylene, and more preferably 1,2-difluoroethylene, from the standpoint of more easily improving stability.

1,2-Difluoroethylene has two types of isomers (E-isomer and Z-isomer). Specifically, there are two types of isomers: trans-1,2-difluoroethylene (HFO-1132(E)) and cis-1,2-difluoroethylene (HFO-1132(Z)). In the fluoroethylene composition of the present disclosure, when fluoroethylene A is 1,2-difluoroethylene, the 1,2-difluoroethylene may be one of these isomers or a mixture of both isomers. That is, when fluoroethylene A is 1,2-difluoroethylene in the fluoroethylene composition of the present disclosure, the 1,2-difluoroethylene is trans-1,2-difluoroethylene (HFO-1132(E)) and/or cis-1,2-difluoroethylene (HFO-1132(Z)).

When fluoroethylene A is 1,2-difluoroethylene, the 1,2-difluoroethylene is preferably its E-isomer. Specifically, the 1,2-difluoroethylene preferably contains 50 mass% or more, more preferably 55 mass% or more, still more preferably 60 mass% or more, and particularly preferably 70 mass% or more of the E-isomer.

In the present specification, the expression "1,2-difluoroethylene" or "HFO-1132" includes all of the following: the E-isomer alone of 1,2-difluoroethylene, the Z-isomer alone of 1,2-difluoroethylene, and a mixture of the E-isomer and Z-isomer of 1,2-difluoroethylene. In the present specification, the E-isomer alone of 1,2-difluoroethylene is referred to as "1,2-difluoroethylene(E)" or "HFO-1132(E)," the Z-isomer alone of 1,2-difluoroethylene is referred to as "1,2-difluoroethylene(Z)" or "HFO-1132(Z)," and a mixture of the E-isomer and Z-isomer of 1,2-difluoroethylene is referred to as "1,2-difluoroethylene(E,Z)" or "HFO-1132(E,Z)," where necessary.

In the fluoroethylene composition of the present disclosure, the production method for fluoroethylene A may be any method. For example, fluoroethylene A can be produced by known production methods. 1,2-Difluoroethylene for use as fluoroethylene A can be produced by a reaction of dehydrofluorination of 1,1,2-trifluoroethane, hydrogenation of 1,2-dichloro-1,2-difluoroethylene, hydrogenation and decomposition of 1,2-dichlorodifluoroethylene, or dehydrochlorination of 1-chloro-1,2-difluoroethane.

The fluoroethylene composition of the present disclosure has a water content of 100 mass ppm or less based on the mass of fluoroethylene A. This improves the stability of fluoroethylene A. If the fluoroethylene composition of the present disclosure has a water content exceeding 100 mass ppm based on the mass of fluoroethylene A, solids will be generated due to a side reaction etc., which deteriorates the stability of fluoroethylene instead.

The fluoroethylene composition has a water content of preferably 0.1 mass ppm or more, more preferably 1 mass ppm or more, still more preferably 2 mass ppm or more, even more preferably 3 mass ppm or more, particularly preferably 4 mass ppm or more, and most preferably 5 mass ppm or more, based on the mass of fluoroethylene A. As long as the fluoroethylene composition has a water content of 100 mass ppm or less, the stability is not easily deteriorated. However, from the standpoint of further preventing the formation of agglomerates etc. described later, the water content is preferably 80 mass ppm or less, more preferably 60 mass ppm or less, still more preferably 50 mass ppm or less, and particularly preferably 20 mass ppm or less, based on the mass of fluoroethylene A. Further, the water content may also be 9 mass ppm or less or 5 mass ppm or less based on the mass of fluoroethylene A.

The water content in the fluoroethylene composition can be measured with a commercially available Karl Fischer moisture measurement apparatus; the detection limit is typically 0.1 mass ppm. Further, the total amount of fluoroethylene A in the fluoroethylene composition can be identified by gas chromatography. Therefore, a water content based on the mass of fluoroethylene A can be measured by using a gas chromatograph and a moisture measurement apparatus in combination.

The fluoroethylene composition contains fluoroethylene A and water, whereby the stability of fluoroethylene A improves, and solids, such as agglomerates, are less likely to be generated even after long-term storage. Although no limited interpretation is desired, the following mechanism can be established as the reason that the presence of water improves the stability of fluoroethylene A. First, water present in the fluoroethylene composition reacts with or stabilizes fluorine-containing methyl radicals or fluorine-containing ethyl radicals generated in the initial stage of decomposition of fluoroethylene A. This makes the reaction between fluoroethylene A and the radicals generated in the initial stage of decomposition less likely to occur, or reduces the reaction rate, making chain reactions, oxidation, etc. less likely to occur, thus improving the stability of fluoroethylene A. On the other hand, if the water content exceeds a certain level (exceeding 100 mass ppm), fluoroethylene A and the fluorine-containing methyl radicals or fluorine-containing ethyl radicals dissolve into water, and so the concentration becomes locally high, and the water forms a reaction field. This makes a polymerization reaction of fluoroethylene A occur, and fluoroethylene A is thus more likely to be consumed, causing a reduction in the stability of fluoroethylene A, as well as causing the formation of agglomerates resulting from the polymerization reaction.

The fluoroethylene composition further contains oxygen. The oxygen content (proportion) is 0.35 mol% or less based on the number of moles of fluoroethylene A. The oxygen content in the fluoroethylene composition means the content of oxygen present in the liquid phase when the fluoroethylene composition is sealed in a container.

The fluoroethylene composition has an oxygen content of more preferably 0.12 mol% or less based on the number of moles of fluoroethylene A. The fluoroethylene composition has an oxygen content of preferably 0.0001 mol% or more, and more preferably 0.001 mol% or more, based on the number of moles of fluoroethylene A. An oxygen content within these ranges improves the stability of fluoroethylene in the composition. From this standpoint, the lower the oxygen content in the composition, the more preferable; however, since the composition contains water as stated above, even when the oxygen content is within the above ranges, the stability of haloolefins can be retained due to the action of the water. More specifically, although the presence of oxygen generally destabilizes fluoroethylene A, water contained in the fluoroethylene composition of the present disclosure prevents adverse effects that can be caused by oxygen on fluoroethylene A. Accordingly, even though the fluoroethylene composition of the present disclosure contains oxygen, fluoroethylene A exhibits excellent stability.

The oxygen content in the fluoroethylene composition can be quantified by measuring the oxygen content in the gas phase by using a commercially available gas chromatograph and converting the measured value into the oxygen content in the liquid phase. The detection limit is typically less than 0.0001 mol%.

The fluoroethylene composition may contain compounds other than fluoroethylene A. For example, the fluoroethylene composition may contain impurities that can become mixed in during the production of fluoroethylene A. When fluoroethylene A is 1,2-difluoroethylene, examples of such impurities include at least one member of hydrogen fluoride, fluoroethylene, trifluoroethylene, 1,1,1-trifluoroethane, propylene, acetylene, difluoromethane, trifluoromethane, fluoromethane, 1,1,2-trifluoroethylene (HFO-1123), 1,1-difluoroethane (HFC-152a), fluoroethane (HFC-161), 1,1,2-trifluoroethane (HFC-143), 2-chloro-1,1,1-trifluoroethane (HCFC-133b), 1-chloro-1,1,2-trifluoroethane (HCFC-133), 1,1-dichloro-2,2,2-trifluoroethane (HCFC-123), 1-chloro-1,2-difluoroethane (HCFC-142a), 1,2-difluoroethane (HFC-152), chlorodifluoromethane (HCFC-22), 1,1,1,2-tetrafluoroethane (HFC-134a), 1,1,2,2-tetrafluoroethane (HFC-134), pentafluoroethane (HFC-125), 2,3,3,3-tetrafluoropropene (HFO-1234yf), 1,2,3,3,3-pentafluoropropene (HFO-1225ye), 1,3,3,3-tetrafluoropropene (HFO-1234ze), 1,3,3,3-tetrafluoropropene (HFO-1234ze), fluoroethylene (HFO-1141), 3,3,3-trifluoropropene (HFO-1243zf), 1,1-difluoroethylene (HFO-1132a), 1-chloro-2,2-difluoroethylene (HCFO-1122), 1-chloro-1,2-difluoroethylene (HCFO-1122a), ethylene, and the like.

When the fluoroethylene composition contains the impurities mentioned above, their content is not limited. For example, the impurities are preferably contained in an amount of 0.1 mass ppm or more, and 10000 mass ppm or less, based on the mass of fluoroethylene A. Within this range, the stabilizing effect on fluoroethylene A is less likely to be interfered with.

In addition to fluoroethylene A and the impurities mentioned above, the fluoroethylene composition may contain other fluorine compounds as long as the effects of the fluoroethylene composition of the present disclosure are not impaired. Examples of the fluorine compounds include various known refrigerants, including haloolefin compounds having 2 to 4 carbon atoms, preferably 2 to 3 carbon atoms, and still more preferably 3 carbon atoms.

When the fluoroethylene composition of the present disclosure is used for a refrigerant or a heat transfer medium, the fluoroethylene composition may contain a lubricating oil. The lubricating oil is not limited. For example, known lubricating oils used for refrigerants etc. can be widely used.

Examples of specific lubricants include at least one member selected from the group consisting of polyalkylene glycols, polyol esters, and polyvinyl ethers. Examples of polyalkylene glycol (PAG) include SUNICE P56, produced by Japan Sun Oil Company, Ltd. Examples of polyol ester (POE) include Ze-GLES RB32, produced by JX Nippon Oil & Energy Corporation.

The lubricating oil may be contained in an amount of 1 to 50 mass%, and preferably 10 to 40 mass%, based on the total amount of fluoroethylene A, water, and the impurities contained in the composition. However, the amount is not limited to these ranges, and can vary depending on the specifications of the oil tank of a refrigerating machine.

In addition to the above, the fluoroethylene composition of the present disclosure may further contain other additives (except for the lubricants mentioned above) or inevitably contained elements, components of compounds, etc. (referred to below as "other components"). When the fluoroethylene composition contains other components, the content of the other components may be 5 mass% or less, preferably 1 mass% or less, more preferably 0.1 mass% or less, and particularly preferably 0.05 mass% or less, based on the mass of fluoroethylene A. The proportion of fluoroethylene A may be 50 mass% or more, preferably 60 mass% or more, more preferably 70 mass% or more, still more preferably 80 mass% or more, and particularly preferably 90 mass% or more, based on the mass of all of the fluorine compounds (fluoro-group-containing compounds) contained in the fluoroethylene composition. The proportion of fluoroethylene A may also be 99 mass% or more based on the mass of all of the fluorine compounds contained in the fluoroethylene composition.

The fluoroethylene composition of the present disclosure can be used for various applications and suitably used for heat transfer media, refrigerants, foaming agents, solvents, cleaning agents, propellants, fire extinguishers, and the like. When the fluoroethylene composition of the present disclosure is present, the quality of heat transfer media, refrigerants, foaming agents, solvents, cleaning agents, propellants, fire extinguishers, and the like is maintained over a long period of time. These heat transfer media, refrigerants, foaming agents, solvents, cleaning agents, propellants, fire extinguishers, and the like can have the same structure as that of known products, except for containing the fluoroethylene composition of the present disclosure.

The fluoroethylene composition of the present disclosure can also be applied to various heat cycle systems. Examples of heat cycle systems include various air conditioners, such as room air conditioners, packaged air conditioners for stores, packaged air conditioners for buildings, packaged air conditioners for facilities, separate air conditioners connected with one or more indoor units and outdoor units through a refrigerant pipe, window air conditioners, portable air conditioners, rooftop or central air conditioners for sending cool or warm air through a duct, and automobile air conditioners; gas engine heat pumps; air conditioners, such as air conditioners for trains and air conditioners for automobiles; showcases, such as built-in showcases and separate showcases; various refrigerating machines, such as refrigerator freezers for businesses, ice machines, integrated refrigerating machines, vending machines, refrigerating machines for cooling containers or refrigerators such as for marine shipping, chiller units, and turbo refrigerating machines; and apparatuses exclusively used for a heating cycle. Examples of apparatuses exclusively used for a heating cycle include water-heating devices, floor-heating devices, and snow-melting devices.

The method of preparing the fluoroethylene composition of the present disclosure is not limited. For example, the fluoroethylene composition of the present disclosure can be prepared by mixing fluoroethylene A and water in a predetermined ratio. In the mixing, the lubricating oil and/or other additives mentioned above can also be appropriately added. Further, by blowing air or oxygen into the fluoroethylene composition, the amount of oxygen in the fluoroethylene composition can be adjusted to be within a desired range.

On the other hand, in the production of fluoroethylene A, water may coexist with fluoroethylene A. When such fluoroethylene A is used, (1) the fluoroethylene composition of the present disclosure can be produced by mixing water and fluoroethylene A, (2) the fluoroethylene composition of the present disclosure can be produced by maintaining the amount of water, or (3) the fluoroethylene composition of the present disclosure can be produced by reducing the amount of water. The method of reducing the amount of water is not limited, and any known method can be widely used. Examples include a drying method that uses an adsorbent.

In the production of fluoroethylene A, the impurities mentioned above may coexist with fluoroethylene A. These impurities may be removed in advance by an appropriate method before the preparation of the fluoroethylene composition, or may otherwise be used without removal to prepare the fluoroethylene composition.

Methods comprising adjusting the content of the water to 100 mass ppm or less based on the mass of the fluoroethylene as described in (1) to (3) above are suitable for stabilizing fluoroethylene A.

### Examples

The present disclosure is described in more detail below with reference to Examples. However, the present disclosure is not limited to the embodiments of the Examples.

### Examples

1,2-Difluoroethylene was produced by dehydrofluorination of 1,1,2-trifluoroethane (HFC-143). The obtained 1,2-difluoroethylene was dehydrated by, for example, allowing it to flow through a porous polymer. The water content in 1,2-difluoroethylene after treatment was measured with a Karl Fischer moisture measurement apparatus, which confirmed that no water was detected. The 1,2-difluoroethylene contained 60% of E-isomer and 40% of Z-isomer. Furthermore, the 1,2-difluoroethylene contained the following impurities:
1,1,1-trifluoroethane (HFC-143a),
1,1,2-trifluoroethylene (HFO-1123),
1,1-difluoroethane (HFC-152a),
fluoroethane (HFC-161),
1,1,2-trifluoroethane (HFC-143),
2-chloro-l,1,1-trifluoroethane (HCFC-133b),
1-chloro-1,1,2-trifluoroethane (HCFC-133),
1,1-dichloro-2,2,2-trifluoroethane (HCFC-123),
1-chloro-1,2-difluoroethane (HCFC-142a),
1,2-difluoroethane (HFC-152),
difluoromethane (HFC-32),
fluoromethane (HFC-41),
chlorodifluoromethane (HCFC-22),
1,1,1,2-tetrafluoroethane (HFC-134a),
1,1,2,2-tetrafluoroethane (HFC-134),
pentafluoroethane (HFC-125),
2,3,3,3-tetrafluoropropene (HFO-1234yf),
1,2,3,3,3-pentafluoropropene (HFO-1225ye),
1,3,3,3-tetrafluoropropene (HFO-1234ze),
fluoroethylene (HFO-1141),
3,3,3-trifluoropropene (HFO-1243zf),
1,1-difluoroethylene (HFO-1132a),
1-chloro-2,2-difluoroethylene (HCFO-1122),
1-chloro-1,2-difluoroethylene (HCFO-1122a),
ethylene, propylene, and acetylene.

The total amount of these impurities was 10000 mass ppm or less based on the mass of 1,2-difluoroethylene.

Subsequently, a predetermined amount of water was added to the 1,2-difluoroethylene to adjust the water content relative to 1,2-difluoroethylene to be 10, 50, 100, and 1000 mass ppm. The 1,2-difluoroethylene in which the water content was adjusted accordingly was sealed in a container, and oxygen was blown into the container to adjust the amount of oxygen relative to 1,2-difluoroethylene as shown in Table 1, based on 1,2-difluoroethylene. In this manner, fluoroethylene compositions in which the water content and oxygen content were adjusted to predetermined proportions as shown in Table 1 (Nos. 7 to 9, 12 to 14, and 17 to 19) were obtained.

### Comparative Examples

Fluoroethylene compositions that contained no water and in which the oxygen amount was adjusted to a predetermined proportion as shown in Table 1 were obtained in the same manner as in the Examples, except that no water was added or no oxygen was added to 1,2-difluoroethylene used in the Examples.

### Evaluation Method

### Fluoroethylene Stability Test 1

Each of the fluoroethylene compositions obtained in the Examples and Comparative Examples above was subjected to a stability test as follows. Specifically, each of the fluoroethylene compositions was placed in a glass tube (8 mm ID × 12 mm OD × 300 mm L) with one side sealed by fusing, so that the content of 1,2-difluoroethylene was 0.01 mol. The tube was then hermetically sealed by fusing. The tube was allowed to stand in a constant-temperature bath in an atmosphere at 150°C and kept in this state for one week. Thereafter, the tube was removed from the constant-temperature bath and cooled. While the appearance was observed, the acid in the gas inside the tube was analyzed to thus evaluate the stability of fluoroethylene.

In fluoroethylene stability test 1, the acid in the gas was analyzed by the following method. Gas remaining in the above tube after cooling was completely solidified using liquid nitrogen. The tube was then opened and gradually thawed to collect gas into a Tedlar bag. Five grams of pure water was injected into the Tedlar bag to extract the acid into the pure water while efficiently bringing pure water into contact with the collected gas. The extract was detected by ion chromatography to determine the content (mass ppm) of fluoride ions (F⁻).

**Table 1**

| No. | Example/ Comparative Example | Oxygen content (mol%) | Water content (mass ppm) | Acid content (mass ppm) | Appearance (presence of solids) |
|---|---|---|---|---|---|
| 1 | Ref. Ex. | 0 | 0 | <1 | No |
| 2 | Ref. Ex. | 0 | 10 | <1 | No |
| 3 | Ref. Ex. | 0 | 50 | <1 | No |
| 4 | Ref. Ex. | 0 | 100 | <1 | No |
| 5 | Ref. Ex. | 0 | 1000 | <1 | Yes |
| 6 | Comp. Ex. | 0.010 | 0 | 100 | No |
| 7 | Ex. | 0.010 | 10 | 50 | No |
| 8 | Ex. | 0.010 | 50 | 40 | No |
| 9 | Ex. | 0.010 | 100 | 10 | No |
| 10 | Comp. Ex. | 0.010 | 1000 | <1 | Yes |
| 11 | Comp. Ex. | 0.015 | 0 | 400 | No |
| 12 | Ex. | 0.015 | 10 | 200 | No |
| 13 | Ex. | 0.015 | 50 | 100 | No |
| 14 | Ex. | 0.015 | 100 | 50 | No |
| 15 | Comp. Ex. | 0.015 | 1000 | 20 | Yes |
| 16 | Comp. Ex. | 0.345 | 0 | 1000 | No |
| 17 | Ex. | 0.345 | 10 | 700 | No |
| 18 | Ex. | 0.345 | 50 | 400 | No |
| 19 | Ex. | 0.345 | 100 | 100 | No |
| 20 | Comp. Ex. | 0.345 | 1000 | 50 | Yes |

Table 1 shows the results of fluoroethylene stability Test 1. In the Reference Examples, in which the compositions contained no oxygen (below the detection limit), fluoroethylene was stable with or without water. However, in No. 5 in Table 1, in which the composition contained too much water, the generation of solids was observed in the composition even in the absence of oxygen.

In all of the compositions obtained in the Examples, the generation of acid was suppressed, fluoroethylene was stable, and additionally, no solids were visually observed. In contrast, in the compositions with a water content of 0 ppm, a large amount of acid was generated, confirming the decomposition of fluoroethylene. In contrast, in the compositions of Nos. 10, 15, and 20, which contained too much water, although the decomposition of fluoroethylene was less likely to occur, the generation of solids was observed.

The above results clarify that a specific amount of water contained in the compositions stabilizes 1,2-difluoroethylene. This indicates that the above compositions can exhibit excellent performance for heat transfer medium, refrigerants, foaming agents, solvents, cleaning agents, propellants, and fire extinguishers, and that the performance is stably maintained. Therefore, the above compositions are considered to be suitable for use with heat transfer medium, refrigerants, foaming agents, solvents, cleaning agents, propellants, and fire extinguishers.

## Claims

1. A fluoroethylene composition containing a fluoroethylene having one or more fluorine atoms, water, and oxygen,
the composition having a water content of 100 mass ppm or less based on the mass of the fluoroethylene, and an oxygen content of 0.35 mol% or less based on the fluoroethylene.

2. The fluoroethylene composition according to claim 1, wherein the fluoroethylene is at least one member selected from the group consisting of 1,2-difluoroethylene and trifluoroethylene.

3. The fluoroethylene composition according to claim 2, wherein the fluoroethylene is 1,2-difluoroethylene.

4. The fluoroethylene composition according to claim 2 or 3, wherein the 1,2-difluoroethylene is trans-1,2-difluoroethylene (HFO-1132(E)) and/or cis-1,2-difluoroethylene (HFO-1132(Z)).

5. The fluoroethylene composition according to any one of claims 1 to 4, further comprising at least one member selected from the group consisting of polyalkylene glycols, polyol esters, and polyvinyl ethers.

6. The fluoroethylene composition according to any one of claims 1 to 5, which is used for a heat transfer medium, a refrigerant, a foaming agent, a solvent, a cleaning agent, a propellant, or a fire extinguisher.
